(19) Europäisches Patentamt
European Patent Office
Office européen des brevets

(11) Publication number: **0 091 383**
**B1**

(12) # EUROPEAN PATENT SPECIFICATION

(45) Date of publication of patent specification: **04.03.87**

(21) Application number: **83400707.2**

(22) Date of filing: **07.04.83**

(51) Int. Cl.⁴: **C 07 C 85/11**, C 07 C 87/50, C 07 C 109/04

(54) **Process for the catalytic reduction of nitro-aromatic compounds.**

(30) Priority: **09.04.82 IT 2066982**

(43) Date of publication of application:
**12.10.83 Bulletin 83/41**

(45) Publication of the grant of the patent:
**04.03.87 Bulletin 87/10**

(84) Designated Contracting States:
**BE DE FR GB NL**

(56) References cited:
**DE-A-3 008 671**
**FR-A-2 211 448**
**US-A-3 906 045**

**CHEMICAL ABSTRACTS, vol. 91, no. 11, 10th September 1979, page 746, no. 91268m, Columbus, Ohio, USA V.Z. SHARF et al.: "Reduction of nitrobenzene by 2-propanol in the presence of tris(triphenylphosphine)rhodium (I) chloride and tris(triphenylphosphine)ruthenium (II) chloride"**

(73) Proprietor: **Montedison S.p.A.**
**31, Foro Buonaparte**
**I-20121 Milan (IT)**

(72) Inventor: **Mestroni, Giovanni**
**27 Vicolo Castagneto**
**Trieste (IT)**
Inventor: **Zassinovich, Grazia**
**27 Vicolo Castagneto**
**Trieste (IT)**
Inventor: **Camus, Annamaria**
**6 Via Hermet**
**Trieste (IT)**
Inventor: **Del Bianco, Clorinda**
**8 Via E Maggiore**
**S. Martino Al Tagliamento (Pordenone) (IT)**

(74) Representative: **Hirsch, Marc-Roger**
**Cabinet Hirsch 34 rue de Bassano**
**F-75008 Paris (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

(56) References cited:

**CHEMICAL ABSTRACTS, vol. 95, no. 1, 6th July 1981, page 632, no. 6668g, Columbus, Ohio, USA, S.I. KONDRAT'EV et al.: "Preparation of chloro-substituted anilines in the presence of a homogeneous catalytic system based on a complex of iridium with chloranilic acid"**

**CHEMICAL ABSTRACTS, vol. 98, no. 17, 25th April 1983, page 522, no. 142760v, Columbus, Ohio, USA, G. MESTRONI et al.: "Selective reduction of aromatic nitro compounds via hydrogen transfer catalyzed by iridium (I) complexes with 2,2'-bipyridine, 1,10-phenanthroline and derivatives"**

**Description**

The present invention refers to a process for the catalytic reduction of nitro-aromatic compounds. More particularly, the present invention concerns a catalytic process for the hydrogenation of nitro-aromatic compounds through the transfer of hydrogen from alcohols in the presence of iridium complexes.

The compounds thus obtained consist, besides the aldehyde or the ketone issuing from the primary or secondary de-hydrogenated alcohol, of the derivatives of the partial or total hydrogenation of the starting nitro-aromatic compounds. Said derivatives may, depending on the progressive degree of the conducted hydrogenation and/or on operating conditions, be aryl-hydroxylamines, aryl-hydrazo compounds or aryl-azo compounds.

The products obtained can be used in a wide range of industrial applications. In fact, they represent intermediates for organic syntheses in general, with particular possibilities in the field of fine chemistry. Among these possibilities, aniline has applications in the field of synthetic rubbers (as a vulcanization accelerator and antioxidizing agent), in the production of isocyanates (polyurethanes), and especially, together with other aryl-amines prepared according to the present invention, in the field of the industry of dyestuffs and of the intermediates for photographic and pharmaceutical products, in the field of explosives, plastics, propellants, insecticides, etc.

In their turn, the hydrazo-compounds and the aromatic azo-compounds are used in the field of dyestuffs, etc.

There are known methods for the catalytic reduction of aromatic nitro-derivatives that use as a hydrogenation source both molecular hydrogen and hydrogen-donors, in general alcohols, carbon oxide and water, etc.

In particular there has been described a hydrogenation method or a method of catalytic reduction of nitro-aromatic compounds (phenylic compounds) by means of the transfer of hydrogen from isopropanol, in the presence of phosphinic rhodium and ruthenium complexes and of potassium hydroxide at temperatures greater than 120°C. There are also obtained minor quantities of acrylic hydrazo-derivatives (phenyl derivatives), of azo-derivatives and of azoxy-benzene.

There is an insufficient industrial applicational interest because of the low reaction velocity (rate) of the methods used that leads to low unitary yields.

The catalysts used for this purpose, in fact, produce unsatisfactory reaction rates, lower, even in the order of a hundredfold, than those obtainable by the use of the catalysts according to the present invention, which latter offer much more pronounced characteristics of industrial applicability.

Other methods foresee the use of pressurized molecular hydrogen in the presence of iron complexes, ruthenium and rhodium complexes with phosphines and/or carbon oxide, and of stoichiometric tertiary amines; or else the use of $CO+H_2O$ mixtures under pressure, in the presence of rhodium, iridium, ruthenium, osmium carbonyl clusters at 100°—180°C, in order to obtain aromatic amines.

Furthermore, there have been described aromatic azo-derivatives obtained by the reduction of nitro-aryls with CO under pressure (200 atm) at about 200°C, in the presence of ferro-carbonyls.

The above-mentioned techniques are not, however, directly pertinent to the present invention because molecular hydrogen is used as a reducing agent or, alternatively, CO, possibly in admixture with $H_2O$, under very severe operationing conditions of temperature and pressure. To resume, it only involves methods of a substantially experimental character.

The known methods for industrial processing thus remain the conventional hydrogenation methods with molecular hydrogen and heterogeneous catalysis, carried out using Adams-, nickel de Raney, copper chromite, palladium on carbon catalysts, etc., or with stoichiometric methods with Fe, Zn, Sn and acids (HCl) etc., which do not technologically show elements that are pertinent to the present invention.

According to the present invention, the preparation of hydrogenated compounds (aromatic amines, aryl-hydroxylamines, aryl-azo and aryl-hydrazo compounds), by catalytic transfer of hydrogen from alcohols or glycol donors to the starting nitro-aromatic compounds, with contemporaneous formation of ketones or aldehydes, with the use of particular iridium complexes, proves particularly effective for its selectivity, versatility and, in particular, for the high reaction velocity ensured by the catalysis.

The complex catalysts of iridium used according to the present invention, are compounds known per se and described in the technical literature.

A number of iridium complexes with CO are known in the hydrogenation reaction of nitro-aryls (nitro-benzene) with the use of $CO+H_2O$ as a hydrogen source, i.e., in reactions using in practice only molecular hydrogen as a hydrogenating agent, as indicated above.

In this case it is also a question of a technology other than that adopted in the present invention.

The present invention may, thus, in a certain way represent an unexpected overcoming of the drawbacks of the Prior Art which mentions the use of catalysts based on iridium complexes with CO as described above in reactions of a proper hydrogenation with gaseous $H_2$ or with equivalents $(CO+H_2O)$.

Thus, one object of the present invention is to provide a method for the catalytic reduction of nitro-aromatic compounds, with iridium complexes, which is simple, cheap and particularly selective, especially as to the high catalysis speed, using as hydrogen donors alcohols or glycols.

This and further objects, which will become evident to the man skilled in the art from the following

description, are achieved, according to the present invention, by a process for the catalytic reduction of nitro-aromatic compounds by a transfer reaction of hydrogen from alcohols to nitro-aromatic compounds, catalyzed by iridium complexes, and characterized in that a primary or secondary alcohol having the formula (I) or a glycol:

```
R
 \
  CHOH          (I)
 /
R'
```

is made to react with a nitro-aromatic compound having the formula (II)

$$Ar—(NO_y)_x \qquad (II)$$

wherein:

R and R', also possibly linked to each other to form homo- and heterocycles, indifferently represent either a hydrogen atom or a possibly substituted hydrocarbyl group having up to 30 carbon atoms;

Ar represents either an aryl or a hetero-aryl group, also substituted;

x and y, equal or different from each other, are equal to 1 or 2, in the presence of a complex iridium catalyst chosen from among those having the formulae (III) and (IV):

$$\{Ir\ Chel\ (L—L)\}X \quad (III) \qquad and \qquad Ir\ Chel\ (L)_2Y \quad (IV)$$

wherein:

Chel represents a bivalent nitrogenous compound with a kelating action;

L—L represents a molecule of a diolefine, preferably not conjugated;

L represents a molecule of a mono-olefine;

$X^-$ represents an anion chosen from among $Cl^-$, $Br^-$, $I^-PF_6^-$, $BF_4^-$, $ClO_4^-$, $B(C_6H_5)_4^-$, $OH^-$;

```
R
 \
  CH—O⁻
 /
R'
```

wherein:

R and R' have the significance already given above;

Y represents a halogen, preferably Cl, Br or I; at a temperature comprised between 20°C and the boiling temperature of the reaction mass occurs, preferably in an inert atmosphere and in the optional presence of mineral alkalis.

As indicated above, the symbols R and R' represent a hydrogen atom or a hydrocarbyl group with up to 30 carbon atoms, also substituted with groups inert under reaction conditions. Groups of this type are for instance the esteric, amidic, alcoxylic, cyano, aminic groups.

Analogously, the symbol Ar represents in particular an arylic or hetero-arylic group, possibly condensed, having at least 5 atoms in the carbon ring (arylic groups) or also hetero-atoms, chosen between N, S and O (hetero-arylic groups), as for instance the phenyl, naphtalene, anthracene, thienyl, furane, pyridine, quinoline groups, etc.

Furthermore, there may be present in the Ar group herein above defined, also substituents inert under reaction conditions, chosen, from among those indicated above for the R and R' groups, and also, for instance, from among the alkyl, alkenyl, alkinyl groups, having preferably up to 4 carbon atoms.

Thus, as nitro-aromatic starting compounds of formula (II), as defined above, may be used derivatives belonging to the benzenic, naphtalenic, anthracenic, thienylic, furanic, pirydinic, quinolinic, etc. classes.

From the definition given for the nitro-aromatic compound (II), it appears evident that in case of y=1, the compound itself may in particular be a derivative of the nitrous acid which for convenience, in the context of the description is comprised in what is defined as "nitro"-compounds.

The reaction is usually carried out in the absence of real and proper solvents; either the alcohol of formula (I) or the glycol used in excess acts as a solvent.

At any event, the reaction is compatible with the use of conventional inert solvents such as: toluene, benzene, methanol, $H_2O$ or terbutanol and in admixture with each other.

The use of a solvent, as will be more clearly indicated hereinbelow, may be particularly useful in the case where it is desired to obtain aryl-hydroxylamines or aryl-hydrozo compounds, by means of a partial hydrogenation of the starting nitro-compound (II), at stoichiometric doses of the alcohol donor.

According to one particular aspect of this invention, the hydrogen transfer-reaction may, in fact, be

adjusted in such a way as to obtain, starting from the aromatic nitro-compound (II) or substrate, besides the corresponding amino-derivative, by complete hydrogenation of group or groups NO or $NO_2$ present, also the derivatives corresponding to the partial hydrogenation of the substrate itself, i.e., the corresponding aryl-hydroxylamines, aryl-hydrazo and aryl-azo derivatives, which may be substantially considered intermediate compounds of the total hydrogenation reaction.

The involved hydrogen-transfer reactions may be, in fact, schematically represented by the following equations:

(1)

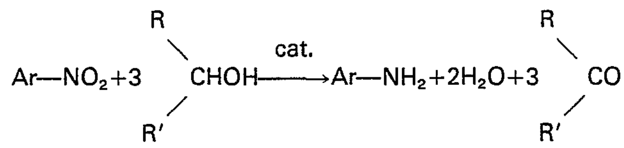


(II) (I)

(2)

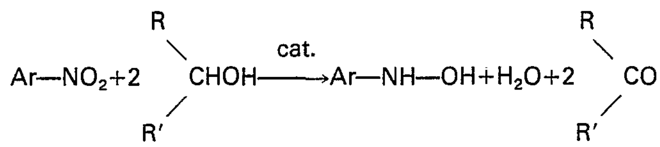


(II) (I) (V)

(3)

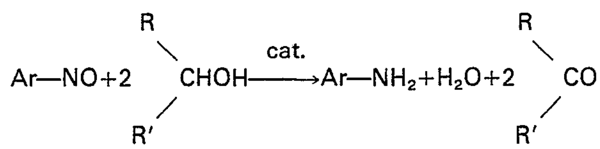


(II) (I)

(4)

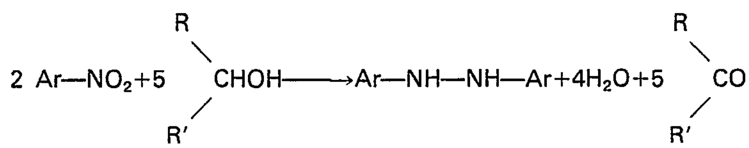

(II) (I) (VI)

(5)

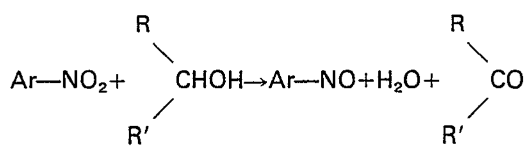

(II) (I) (II)

(6)

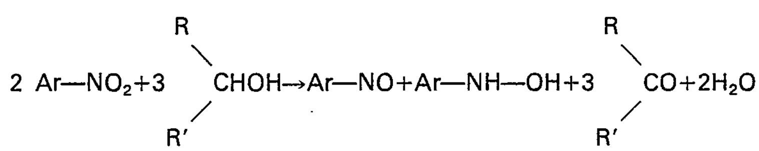

(II) (I) (II) (V)

(7)

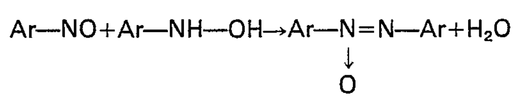

(II) (V) (VII)

(8)

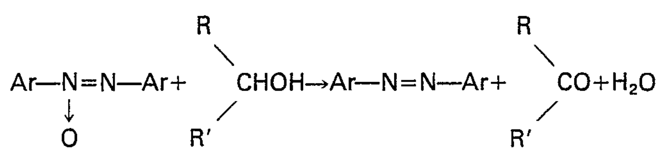

(VII) (I) (VIII)

(9) 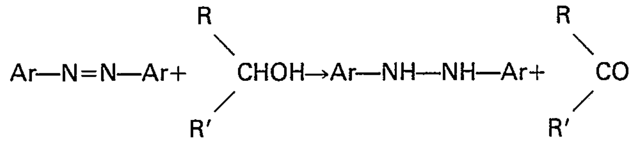

(VIII) (I) (VI)

That is, according to a general scheme for the reactions (1, 2, 4, 5, 6):

(10) 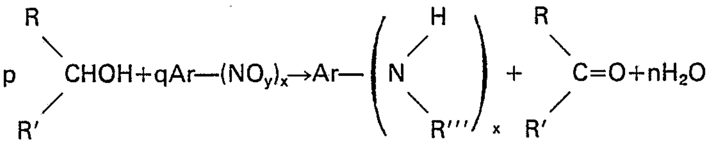

In equation (10), R''' represents H, OH, or for $x=1$, NHAr, while all the other symbols have the significance already indicated hereinabove, and $n$=a whole number or integer from 1 to 4; p is a whole number chosen from among 1, 2, 3, 5; q is a whole number chosen from 1 and 2. Moreover, they have been formulated taking into consideration as a substrate an aromatic nitro-derivative ($y=2$ and $x=1$, in formula (II)), in equations (1), (2), (4), (5) and (6), and a nitrous-aromatic substrate ($y=1$ and $x=1$, in formula (II)), in equation (3).

The whole of these above equations, considered together with equations (3), (8), and (9), quite clearly evidence that, depending on the substrate/alcohol donor molar ratios, it is possible to arrive at the corresponding partial hydrogenation derivatives (equations (2), (4), (5), (6), (7), (8) and (9), or at the total hydrogenation derivatives (equations (1), (3)), but also that the present invention may equally be applied for the hydrogenation of compounds such as: the azoxy-aryls (VII) and the azo-aryls (VIII), partial hydrogenation intermediates of the nitro-aromatic (II) compounds, according to equations (8) and (9), respectively.

To resume, through a suitable choice of the parameters involved, i.e. the molar ratios, the solvent, the quantities of alcohols, the catalyst, etc., it is possible, as will be more clearly indicated hereinunder, to direct the hydrogen transfer reaction in such a way as to obtain the desired hydrogenated derivative.

The catalysts are optionally used in the presence, as indicated above, of small quantities of mineral alkali, preferably chosen from among: NaOH, KOH, LiOH, $Ca(OH)_2$, and $NaHCO_3$, in a molar ratio preferably comprised between, 0.1 and 200 with respect to one mole of catalyst.

The choice of the optimal ratio of the mineral alkali with respect to the iridium (III) and (IV) catalyst used, is of considerable importance as concerns the selectively directing of the hydrogen transfer towards the obtention of the desired aminic or hydrazo-product, etc.

Such a choice, moreover, can be made depending on the nature of the starting substrate, the temperature, the molar ratios etc., adopted.

Thus, in particular, in order to selectively obtain the reduction of aromatic nitro-derivatives (II) to the corresponding amines (reactions (1) and (3)), low operational values of the alkaline compound/catalyst molar ratio must be maintained, preferably comprised between 0.5:1 and 1.5:1, and a substrate/catalyst molar ratio, preferably comprised between 5:1 and about 250:1.

Analogously, if it is wished to obtain hydrazo-derivatives (VI) by selective reduction of the corresponding nitro-derivatives (II), of the azoxy- (VII) and of the azo-derivatives (VIII) (equations (4), (8), (9)), it is advisable to operate with high values of above-mentioned molar ratios, and preferably with an alkaline compound/catalyst molar ratio comprised between 10:1 and 100:1, and with a substrate/catalyst molar ratio comprised between 500:1 and 2000:1.

From the hydrazo-derivatives, moreover, the azo-derivatives are obtainable by simple exposure to air.

Lastly, in order to selectively obtain the reduction of nitro-derivatives (II) to the corresponding aryl-hydroxylamines (V) (equations (2) and (6)), it is convenient to operate with low substrate/catalyst molar ratios, preferably at values comprised between 50:1 and 250:1, stopping the reaction as soon as the nitro-aromatic compound has been reduced, while the alkaline compound/catalyst ratio is comprised between 1:1 and 10:1.

Obviously, in these reactions the choice of the catalyst also has its influence, as will be more clearly explained hereinbelow.

In this connection, in order to achieve a more correct dosage of the alcohol donor, it is advisable to operate in a solvent medium different from the alcohol itself.

As far as the catalysts are concerned, the iridium complexes used according to the invention have the formulae (III) and (IV), wherein, in particular, the kelating azotized bi-toothed compound (Chel) is preferably chosen from among: 2,2'-dipyridyl (dipy), 3,3'-dimethyl-2,2'-dipyridyl, 4,4'-dimethyl-2,2'-dipyridyl, 1,10-phenanthroline, 5,6-dimethyl-1,10-phenanthroline, 4,7-dimethyl-1,10-phenanthroline, 3,4,7,8-tetramethyl-1,10-phenanthroline, 4,7-diphenyl-1,10-phenanthroline and sulphonated phenanthroline.

The preferably non-conjugated diolefine (L—L) is chosen from among 1,5-hexadiene, norbornadiene

and 1,5-cyclo-octadiene (cis-cis); the monoolefine (L) is the cyclooctene or ethylene, anion $X^-$ has already been defined.

The following proved to be especially effective catalysts according to the present invention.

. Ir 3,4,7,8$(CH_3)_4$ phen $(CH_2{=}CH_2)_2Cl$;
. Ir phen $(CH_2{=}CH_2)_2Cl$;
. Ir 4,7$(CH_3)_2$ phen $(CH_2{=}CH_2)_2Cl$;
. Ir 4,4'$(CH_3)_2$ dipy $(CH_2{=}CH_2)_2Cl$;
. Ir 4,4'$(C_6H_5)_2$ dipy $(CH_2{=}CH_2)_2Cl$;
. Ir dipy $(CH_2{=}CH_2)_2Cl$;
. Ir 3,4,7,8$(CH_3)_4$ phen COD Cl;
. {Ir 3,4,7,8$(CH_3)_4$ phen (1,5-hexadiene)} $ClO_4$;
. {IR, 4,7$(CH_3)_2$ phen (1,5-hexadiene)} $ClO_4$;
. {Ir phen (1,5-hexadiene)} $ClO_4$;
. {Ir 4,7$(C_6H_5)_2$ phen (1,5-hexadiene)} $ClO_4$;
. {Ir 4,4'$(CH_3)_2$ dipy (1,5-hexadiene)} $ClO_4$;
. {Ir, 4,4'$(C_6H_5)_2$ dipy (1,5-hexadiene)} $ClO_4$;
. {Ir dipy (1,5-hexadiene)} $ClO_4$;

wherein:

"phen" stands for phenanthroline,
"dipy" stands for 2,2'-dipyridyl,
"COD" stands for 1,5-cyclooctadiene.

More particularly, with reference to the obtainable products, particularly effective for the reduction of the aromatic (II) nitro-derivatives to amines and hydrazo-derivatives (VI) have proved to be: Ir 3,4,7,8$(CH_3)_4$phen$(CH_2CH_2)_2Cl$ and {IR-3,4,7,8$(CH_3)_4$phen (1,5-hexadiene)} $ClO_4$; while for obtaining amines and aryl-hydroxylamines (V) the following proved to be particularly effective: Ir 4,4'$(C_6H_5)_2$dipy$(CH_2{=}CH_2)_2Cl$, Ir 4,4'$(C_6H_5)_2$dipy (1,5-hexadiene) $ClO_4$; . . . Ir 4,7$(C_6H_5)_2$phen$(CH_2{=}CH_2)_2Cl$; Ir, 4,7$(C_6H_5)_2$phen(1,5-hexadiene) $ClO_4$; in which:

"phen" stands for pnenanthroline,
"dipy" stands for 2,2-dipyridyl.

Furthermore, catalysts (III) and (IV) provide the maximum catalytic activity provided they are preliminarily subjected to activation through molecular hydrogen and subsequently submitted to reflux-heating. In any event, this is a question of a preferred but not indispensable procedure.

The iridium complexes used as catalysts according to the present invention are in turn prepared according to the techniques known per se.

For instance, the iridium complex having the formula: {IR Chel (L—L)}Cl of type (III), wherein the kelating substance is, for instance, 3,4,7,8-tetramethyl-1,10-phenanthroline and L—L is 1,5-cyclooctadiene (COD), may be prepared starting from a degassed solution of $\{IRCODCL\}_2$ in methylene chloride by the addition of the kelating agent in a slight excess and by successive precipitation with ethyl ether.

The complex catalysts of the iridium of formula (IV) are likewise prepared according to substantially conventional techniques.

For instance, the iridium complex of formula: Ir Chel$(L)_2$Cl of type (IV), wherein 'L' is cyclooctene, is prepared starting from a degassed Ir(cyclooctene) Cl solution in benzene, solution which is then filtered in an inert gas atmosphere and the liquid filtrate is then additioned with an excess of monoolefine (cyclooctene) and then with the azotized kelating agent, precipitating the complexes as solid crystals which are then filtered.

Furthermore, the catalyst may, according to the present invention, also be directly prepared 'in situ' in the reaction medium by the addition of the chosen kelating agent to the halogenated olefine complex of iridium.

The catalyst according to this invention, is used in quantities that may vary within a wide range.

Advantageous results are achieved with the use, for each mole of nitro-aromatic (II) compound, of quantities comprised between $1\times10^{-2}$ and $1\times10^{-4}$ moles of catalyst.

The complex catalysts of the iridium of the invention are used in an optionally basic medium, as specified above, by the presence of mineral alkaline compounds.

Suitable reaction means are preferably the alcoholic solutions constituted by the alcohol donor (I) or by the excess glycol which acts as a reaction medium.

The reaction is conducted according to a molar ratio of the reactants varying within a wide range; practically optimal results are obtainable with values of the alcohol donor/nitro-aromatic acceptor compound molar ratio comprised between 1:1 and 200:1, the alcohol in excess being used as a solvent reaction medium.

The reduction reaction, according to the present invention, is conducted under atmospheric pressure and preferably in an inert atmosphere such as nitrogen, argon, etc.

Temperatures from 20°C to 200°C are possible up to the boiling point of the solution.

Nitro-aromatic reducible compounds according to the present invention are, in particular, among the aromatic substances, nitro-benzene, nitro-toluene, nitro-anisol, nitroaniline, nitro-benzamide, nitrobenzonitrile, nitro-naphtalene, nitro-styrene, dinitro-benzene, dinitro-toluene, dinitro-aniline, dinitrobenzamide, dinitro-benzonitrile; among the hetero-aromatics, the nitro-pyridines, nitro-quinolines, etc. along with the corresponding nitrous-derivatives such as nitrous-benzene, etc., the corresponding aryl-azoxy and aryl-azo compounds.

Thus, compounds (II) are also reducible which have more than one nitric function, which in such a case are preferably reduced using a ratio alkaline compound to a catalyst equal to about 1 and a substrate/catalyst ratio lower than 10.

The isopropylic, ethylic, 2-butylic, cyclohexylic, benzylic, cyclopentylic, etc. alcohols proved to be effective alcohol donors.

The product is then separated according to conventional techniques. Practically it concerns separating the solvent or alcohol possibly in excess by distillation, while the high-boiling fraction consists of the hydrogenated compound, generally in a quantitative way, etc.

More particularly, the amines may thus be isolated after a preliminary filtering the solution under heat, possibly in an inert atmosphere, on animal carbon (coal) for the quantitative separation of the catalyst, by removal of the solvent under vacuum, etc.

The hydrazo-derivatives, for instance hydrazo-benzene, may be obtained by additioning the end solution under heat, and possibly freed from the catalyst as indicated above, under an inert atmosphere, with deaerated water and by removing the solvent.

The aryl-hydroxylamines are obtained by stopping the reaction with an excess of water. During cooling the crystals separate and are then filtered, etc.

The catalyst may in its turn be recovered and recycled, for instance, treating with a solvent (dimethylformamide or dimethylacetamide) the animal carbon into which it is adsorbed by filtration of the reaction mass. By the addition of ether to the solution thus obtained, the catalyst will precipitate and be recycled after having been activated, etc.

Or, alternatively, the final reaction solution, concentrated to a small volume, is diluted with ether until the catalyst begins to precipitate. Thereupon the suspension thus obtained is treated with neutral alumina, by which latter the catalyst remains adsorbed. The catalyst-containing alumina is then filtered, washed with ether and treated with the alcohol donor (isopropanol) and heated while the catalyst is thereafter recycled.

According to an effective form of embodiment, in actual practice, one proceeds as follows.

Into a reactor fitted with reactant-feeding systems and a heat stabilizing device, is introduced, under an inert atmosphere (nitrogen), the possible solvent and the alcohol donor and then the desired quantities of catalyst and the possible base, in a determined ratio. Thereafter one proceeds to the activation of the catalyst and, finally, the nitro-aromatic (II) compound is added, possibly dissolved in alcohol in a pre-fixed ratio, under a nitrogen flow, heating the whole mass at the determined temperature and for the established time.

At the end of the reaction, controlled for instance by gas-chromatography, the separation of the product according to the Prior Art techniques is carried out.

Due to the simple and mild operational conditions, the process is particularly convenient.

A further advantage consists in the fact that it is possible to conduct the selective reduction of the nitro-aromatic (II) compounds also in the presence of olefinic links, an aspect that is of particular industrial interest.

Moreover, the process according to this invention, due to the greater activity of the catalysts with respect to the hydrogen-transferring catalysts of the prior art, allows the obtention of high conversion rates, greater than 98%, in shorter times and with high substrate to catalyst ratios. In particular, they prove to be much more active and selective than the above-mentioned prior art catalysts, when using isopropanol as a hydrogen donor, and in general developing an activity comparable with that of the best reduction catalysts of the prior art. Moreover, these catalysts display a greater versatility with respect to the partial or total hydrogenation products and furthermore they are recoverable.

The invention will now be described in further detail by the following examples, given by way of non-limitative illustration. Examples 43, 44 and 49 are given for by way of comparison with processes of the prior art. The symbols used therein are:

phen=phenanthroline;
"dipy"=2,2'-dipyridyl;
COD=1,5-cyclooctadiene;
ED=1,5-hexadiene;
S=substrate;
C=catalyst;

the ratios are expressed in moles.

Example 1

50 ml of isopropanol are made to reflux in a three-necked flask fitted with a reflux device and connected with an inert gas (argon) supply source. To the degassed solvent was then added the catalyst (11 mg of IR 3,4,7,8$(CH_3)_4$phen$(CH_2{=}CH_2)_2$Cl, $2\times10^{-5}$ moles) and, after 25 minutes, was added the desired quantity (1.12 mg) of KOH dissolved in 0.55 ml of $H_2O$.

After 20 minutes was then added the substrate (0.25 ml of nitrobenzene, $2.4\times10^{-3}$ moles) dissolved in

deaerated isopropanol (1—2 ml) and heated to 65°C. The substrate/catalyst ratio=125, while KOH/catalyst=1.

The course of the reaction is followed through gas-chromatography realizing intermittent drawings. After 12 minutes aniline was obtained with a yield >99%, calculated on the starting nitrobenzene.

Example 2

Operating proceeded as in Example 1, but with adding 0.5 ml of nitrobenzene ($4.88\times10^{-3}$ moles). After 30 minutes an aniline was obtained with a yield greater than 99%.
Ratios: Substrate/catalyst=250; KOH/catalyst=1.

Example 3

Operating proceeded as in Example 1, but with adding 1 ml of nitrobenzene ($9.7\times10^{-3}$ moles). After 90 minutes, aniline was obtained with a yield greater than 99%.
Ratios: S/C=500; KOH/C=1.

Example 4

Operating proceeded in the same way as in Example 1, but with adding 2 ml of nitrobenzene ($1.95\times10^{-2}$ moles).
After 16 hours, an aniline was obtained with a yield greater than 99%.
Ratios: S/C=1000; KOH/C=1.

Example 5

Operating proceeded as in Example 1 and 11 mg of Ir-3,4,7,8$(CH_3)_4$phen$(CH_2{=}CH_2)_2$Cl ($2\times10^{-5}$ moles) were added in an argon flow to 50 ml of reflux-heated isopropanol. After 25 minutes were added to it 2.24 mg of KOH dissolved in 0.55 ml of $H_2O$.
After 20 minutes 1 ml of nitrobenzene ($9.76\times10^{-3}$ moles) was added.
After 45 minutes aniline was obtained with a yield of 90.7% and 9.3% of hydrazobenzene.
Ratios: S/C=500; KOH/C=2.

Example 6

Operating proceeded as in Example 5, and 5.60 mg of KOH were added.
After 20 minutes a yield of 71.9% of aniline and 28.1% of hydrazobenzene were obtained.
Ratios: S/C=500; KOH/C=5.

Example 7

Operating proceeded as in Example 5, and 11.2 mg of KOH were added.
After 15 minutes a yield of 53.1% of aniline and 46.9% of hydrazobenzene were obtained.
Ratios: S/C=500; KOH/C=10.

Example 8

Operating proceeded as in Example 1, and to 50 ml of reflux-heated isopropanol were added 11 mg of Ir 3,4,7,8$(CH_3)_4$phen$(CH_2{=}CH_2)_2$Cl ($2\times10^{-5}$ moles) in a flow of argon. After 25 minutes 11.2 mg of KOH were added. Thereupon 0.25 ml of nitrobenzene were added.
After 10 minutes a yield of 96.3% of aniline and 3.7% of hydrazobenzene were obtained.
Ratios: S/C=125; KOH=10.

Example 9

Operating proceeded as in Example 8, and 0.5 ml of nitrobenzene ($4.88\times10^{-3}$ moles) were added.
After 6 minutes a yield of 79.8% of aniline and of 20.2% of hydrazobenzene were obtained.
Ratios: S/C=250; KOH/C=10.

Example 10

Operating proceeded as in Example 8, and 2 ml of nitrobenzene ($1.95\times10^{-2}$ moles) were added.
After 120 minutes a yield of 29.8% of aniline and of 70.2% of hydrazobenzene were obtained.
Ratios: S/C=1000; KOH/C=10.

Example 11

Operating proceeded as in Example 10, and 22.4 mg of KOH were added.
After 60 minutes yields of 18.8% for the aniline and 81.2% for the hydrazobenzene were obtained.
Ratios: S/C=1000; KOH/C=20.

Example 12

Operating proceeded according to Example 10, and 44.8 mg of KOH were added.
After 60 minutes yields of respectively 13.7% and 86.3% for the aniline and the hydrazobenzene were obtained.
Ratios: S/C=1000; KOH/C=40.

Example 13

Operating proceeded as in Example 10, and 90 mg of KOH were added.

After 60 minutes a yield of 11.7% of aniline and 88.3% of hydrazobenzene were obtained.

After exposure of the solution to air, 88.3% of azobenzene was obtained.

Ratios: S/C=1000; KOH/C=80.

Example 14

Operating proceeded as in Example 1, and to 50 ml of reflux-heated isopropanol, were added in an argon flow 11 mg of Ir 3,4,7,8$(CH_3)_4$phen$(CH_2{=}CH_2)_2$Cl ($2\times10^{-5}$ moles).

After 25 minutes were admixed 11.2 mg of KOH dissolved in 0.55 ml of $H_2O$. To this solution were then admixed 1 ml of nitrobenzene ($9.76\times10^{-3}$ moles).

After 15 minutes a yield of 53.1% of aniline and of 46.9% of hydrazobenzene were obtained.

Ratios: S/C=500; KOH/C=10.

Example 15

Operating proceeded as in Example 14, and using as a catalyst: Ir 4,7$(CH_3)_2$phen$(CH_2{=}CH_2)_2$Cl, after 60 minutes a yield of 62.1% of aniline and one of 37.8% of hydrazobenzene were obtained.

Ratios: S/C=500; KOH/C=10.

Example 16

Operating proceeded as in Example 14, and using as a catalyst: Ir phen$(CH_2{=}CH_2)_2$Cl, after 360 minutes a yield of 40% of aniline and of 60% of hydrazobenzene were obtained.

Ratios: S/C=500; KOH/C=10.

Example 17

Operating proceeded as in Example 14, and using as a catalyst: IR 4,7$(C_6H_5)_2$phen$(CH_2{=}CH_2)_2$Cl, after 10 minutes a yield of 91.6% of aniline and of 8.4% of hydrazobenzene were obtained.

Ratios: S/C=500; KOH/C=10.

Example 18

Operating proceeded as in Example 14, and using as a catalyst: Ir 4,4'$(CH_3)_2$dipy$(CH_2{=}CH_2)_2$Cl, after 60 minutes a yield of 64.2% of aniline and of 33.8% of hydrazobenzene were obtained.

Ratios: S/C=500; KOH/C=10.

Example 19

Operating proceeded as in Example 14, and using as a catalyst: Ir 4,4'$(C_6H_5)_2$dipy$(CH_2{=}CH_2)_2$Cl, after 30 minutes a yield of 88% of aniline and 12% of hydrazobenzene were obtained.

Ratios: S/C=500; KOH/C=10.

Example 20

Operating proceeded as in Example 17 and using as a catalyst 5.6 mg of KOH, after 20 minutes a yield of 93.5% of aniline and of 6.5% of hydrazobenzene were obtained.

Ratios: S/C=500; KOH/C=5.

Example 21

Operating proceeded as in Example 17 and using 2.24 mg of KOH, after 30 minutes a yield of 96.7% of aniline and of 3.3% of hydrazobenzene were obtained.

Ratios: S/C=500; KOH/C=2.

Example 22

Operating as described in Example 1, to 50 ml of reflux-heated isopropanol were added 10 mg of Ir 4,4'$(C_6H_5)_2$dipy$(CH_2{=}CH_2)_2$Cl ($2\times10^{-5}$ moles) in a flow of argon.

After 25 minutes 5.6 mg of KOH were admixed and immediately afterwards 1 ml of nitrobenzene were added. After 30 minutes a yield of 96.5% of aniline and of 3.5% of hydrazobenzene were obtained.

Ratios: S/C=500; KOH/C=5.

Example 23

Operating as described in Example 22, and using 2.24 mg of KOH, after 30 minutes a yield of 98% of aniline and of 2% of hydrazobenzene were obtained.

Ratios: S/C=500; KOH/C=2.

Example 24

Operating as described in Example 22 and using 1.12 mg of KOH, after 41 minutes a yield of 98.6% of aniline and of 1.4% of hydrazobenzene were obtained.

Ratios: S/C=500; KOH/C=1.

Example 25

Proceeding as described in Example 24 and using 2 ml of nitrobenzene, after 360 minutes a yield of 97.3% of aniline and of 2.7% of hydrazobenzene were obtained.

Ratios: S/C=1000; KOH/C=1.

Example 26

Proceeding as described in Example 1, to 50 ml of reflux-heated isopropanol were added in an argon flow 10 mg of Ir $4,4'(C_6H_5)_2dipy(CH_2{=}CH_2)_2Cl$ ($2\times10^{-5}$ moles).

After 25 minutes were added 0.56 mg of KOH in 0.5 ml of $H_2O$ and then were added 0.25 ml of nitrobenzene. After 1 hour, a yield greater than 99% of aniline was obtained.

Ratios: S/C=125; KOH/C=0.5.

Example 27 (for obtaining phenylhydroxylamine)

Proceeding as in Example 1, to 50 ml of reflux-heated isopropanol were added, in an argon flow, 10 mg of Ir $4,4'(C_6H_5)_2dipy(CH_2{=}CH_2)_2Cl$ ($2\times10^{-5}$ moles).

After 25 minutes there were added 1.12 mg of KOH dissolved in 0.55 ml of water ($H_2O$) and immediately after it was admixed with 0.5 ml of nitrobenzene, carrying on the refluxing.

After 3 minutes was obtained 100% conversion of the nitrobenzene to: 90% of phenylhydroxylamine and 10% of aniline.

After 1 hour a yield of more than 99% of aniline was obtained.

Ratios: S/C=250; KOH/C=1.

Example 28

Proceeding as in Example 27 and using 0.25 ml of nitrobenzene, after 3 minutes was obtained a 100% conversion of the nitrobenzene to: >90% of phenylhydroxylamine and to <10% aniline.

After 1 hour a yield of >99% of aniline was obtained.

Ratios: S/C=125; KOH/C=1.

Example 29 (reduction of azoxybenzene)

Proceeding as described in Example 1, to 50 ml of reflux-heated isopropanol were added in an argon flow 11 mg of Ir $3,4,7,8(CH_3)_4phen(CH_2{=}CH_2)_2Cl$ ($2\times10^{-5}$ moles).

After 25 minutes were added 2.24 mg of KOH dissolved in 0.55 ml of $H_2O$ and then 1 g of azoxybenzene dissolved at 65°C in a minimum quantity of isopropanol.

After 10 minutes there was obtained a 100% conversion of the azoxybenzene to >99% of hydrazobenzene.

Ratios: S/C=252; KOH/C=2.

Example 30 (reduction of the azobenzene)

Operating proceeded as in Example 29, using 1 g of azobenzene.

After 5 minutes a yield of >99% of hydrazobenzene was obtained.

Ratios: S/C=274; KOH/C=2.

Example 31 (reduction of azoxybenzene)

Operating proceeded as in Example 29, using as a catalyst: Ir $4,4'(C_6H_5)_2dipy(CH_2{=}CH_2)_2Cl$.

After 10 minutes there was obtained a 100% conversion of the azooxybenzene to >99% of hydrazobenzene.

Ratios: S/C=252; KOH/C=2.

Example 32

Proceeding as described in Example 1, to 50 ml of reflux-heated isopropanol, were added 11 mg of Ir $3,4,7,8(CH_3)_4phen(CH_2{=}CH_2)_2Cl$ ($2\times10^{-5}$ moles) in an argon current.

After 25 minutes were added 1.12 mg of KOH dissolved in 0.55 ml of $H_2O$. To this solution there were then additioned 0.5 g of p-nitrotoluene ($3.3\times10^{-3}$ moles).

After 10 minutes there was obtained a yield of more than 99% of p-aminotoluene.

Ratios: S/C=165; KOH/C=1.

Example 33

Operating as described in Example 32 and using 0.7 g of 2-nitronaphtalene, after 15 minutes there was obtained a yield equal to 99% of 2-aminonaphtalene.

Ratios: S/C=200; KOH/C=1.

Example 34

Operating as described in Example 32 and using 0.5 g of p-nitroanisol, after 10 minutes, there was obtained a yield >99% of p-amino-anisol.

Ratios: S/C=163; KOH/C=1.

Example 35

Operating as described in Example 1, to 50 ml of isopropanol, reflux-heated, were added 22 mg of Ir 3,4,7,8$(CH_3)_4$phen$(CH_2{=}CH_2)_2$Cl ($4\times10^{-5}$ moles) in an argon flow.

After 25 minutes 2.4 mg of KOH were added and subsequently 200 mg of 2,4-dinitrotoluene. After 30 minutes a yield of more than 99% of 2,4-diamino-toluene was obtained.

Ratios: S/C=5; KOH/C=1.

Example 36

Operating proceeded as described in Example 1, to 50 ml of a reflux-heated sec-butanol in an argon flow 11 mg of Ir 3,4,7,8$(CH_3)_4$phen$(CH_2{=}CH_2)_2$Cl were added.

After 20 minutes 0.55 ml of $H_2O$ containing 1.12 mg of KOH were added. Thereupon 1 ml of nitrobenzene were added.

After 120 minutes a yield of >99% of aniline was obtained.

Ratios: S/C=500; KOH/C=1.

Example 37 (recovery and recycling of the catalyst)

The end solution obtained in Example 36, after separation of the aniline, concentrated to a small volume by recovering the solvent and the alcohol donor, was diluted with ether until the beginning of the precipitation of the catalyst.

The suspension thus obtained was then treated with neutral alumina.

The catalyst was then adsorbed on the alumina and the catalyst-containing alumina was then filtered, washed with ether, treated with 50 ml of isopropanol and reflux-heated.

Catalyst IR 3,4,7,8$(CH_3)_4$phen$(CH_2{=}CH_2)_2$Cl, recovered according to the above indicated procedure, was then treated with 1.5 mg of KOH and then with 0.25 ml of nitrobenzene (2nd production cycle).

After 16 hours under refluxing, a yield of 70% of aniline and 30% of hydrazobenzene was obtained.

Example 38 (recovery and recycling of catalyst)

The IR 4,7$(C_6H_5)_2$phen$(CH_2{=}CH_2)_2$Cl catalyst, recovered from Example 17 according to the general procedure of Example 37, was treated with 5 mg of KOH and then with 0.25 ml of nitrobenzene.

After 6 hours a yield of 47% of aniline and of 53% of hydrazobenzene was obtained.

Example 39 (use of oxidized catalyst)

11 mg of Ir 3,4,7,8$(CH_3)_4$phen COD Cl were dissolved in 50 ml of isopropanol and oxidized in air at room temperature for 12 hours. Operating then proceeded as described in Example 1. To the degassed and reflux-heated solution were added 1.12 mg of KOH dissolved in 0.6 mg of $H_2O$. After 30 minutes 1 ml of nitrobenzene was then added.

After 8 hours a yield of 91% of aniline and 9% of hydrazobenzene was obtained.

Ratios: S/C=500; KOH/C=1.

Example 40

Operating proceeded as in Example 39, and 11.3 mg of Ir 4,4'$(C_6H_5)_2$dipy$(CH_2{=}CH_2)_2$Cl were used.

After 30 minutes a yield equal to 98.2% of aniline and 1.8% of hydrazobenzene was obtained.

Ratios: S/C=500; KOH/C=1.

Example 41 (reduction of nitrobenzene in the absence of KOH)

To 50 ml of a reflux-heated cyclo-pentanol-$H_2O$ (3% $H_2O$) were added in an atmosphere of argon gas, 11 mg of Ir 3,4,7,8$(CH_3)_4$phen$(CH_2{=}CH_2)_2$Cl ($2\times10^{-5}$ moles). Then operating as described in Example 1, 0.25 ml of nitrobenzene were added.

After 2 hours a yield equal to 7% of aniline was obtained.

Example 42

Operating proceeded as described in Example 41, and as catalyst Ir 4,4'$(C_6H_5)_2$dipy$(CH_2{=}CH_2)_2$Cl was used.

After 4 hours a yield of 11.2% of aniline was obtained.

Examples 43 and 44 (for comparison)

Operating proceeded as described in Example 1, the reduction of nitrobenzene, by using Ir 3,4,7,8$(CH_3)_4$phen$(CH_2{=}CH_2)_2$Cl as a catalyst, according to ratios indicated in Example 2, and by the subsequent substitution of the preceding catalyst with catalyst RhCl$\{P(C_6H_5)_3\}_3$ of the prior art, at the optimal condition therein indicated, led to the following results as recorded on the table herein-under.

TABLE

| Catalyst | Alcohol donor | Ratio S/cat. | Tempera-ture °C | % Conver-sion | % Aniline | Time |
|---|---|---|---|---|---|---|
| $RhCl\{P(C_6H_5)_3\}_3$ | 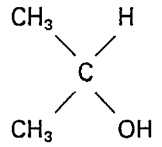 | 10 | 180 | 100 | 80 | 2 hrs |
| Ir $3,4,7,8(CH_3)_4$phen-$(CH_2{=}CH_2)_2Cl$ | 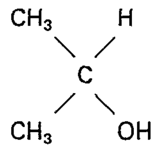 | 250 | 83 | >99 | >99 | 30 min |

Example 45

Into a three-necked flask with a refluxing device, were placed refluxing 50 ml of isopropanol, the refluxing device being connected with a flow of an inert gas (argon). To the deaerated or degassed solvent the catalyst (12 mg of {Ir $3,4,7,8(CH_3)_4$phen ED}$ClO_4$, $2\times10^{-5}$ moles) (ED=1,5-hexadiene) was added.

The solution was then saturated with hydrogen and after 10 minutes the saturated solution was reflux heated.

Thereupon it was admixed with the desired quantity (1.12 mg) of KOH dissolved in 0.55 ml of $H_2O$.

After 15 minutes was then added the substrate (0.50 ml of nitrobenzene, $4.88\times10^{-3}$ moles) dissolved in deaerated isopropanol (1—2 ml) where after it was heated to 65°C.

Ratios: S/C=250; KOH/C=1.

The course of the reaction was followed through gas chromatography, taking drawings at established time intervals. After 15 minutes aniline was obtained with a yield of >99%, calculated on the starting nitrobenzene.

Example 46

Operating proceeded as described in Example 45, adding 1 ml of nitrobenzene ($9.76\times10^{-3}$ moles).

After 30 minutes aniline with a yield of 98.5% was obtained.

Ratios: S/C=500; KOH/C=1.

Example 47

Operating proceeded as in Example 45, but by adding 2 ml of nitrobenzene ($1.95\times10^{-2}$ moles).

After 180 minutes aniline with a yield of 98.5% was obtained.

Ratios: S/C=1,000; KOH/C=1.

Example 48

Operating proceeded as described in Example 45, but adding $1\times10^{-5}$ moles of {Ir $3,4,7,8(CH_3)_4$phen ED}$ClO_4$ and 0.5 g of p-nitro-toluene ($3.65\times10^{-3}$ moles).

After 15 minutes there was obtained p-toluidine with a yield of >99%.

Ratios: S/C=360; KOH/C=1.

When successively substituting the preceding catalyst with $RhCl\{P(C_6H_5)_3\}_3$ of the prior art, 1% of p-toluidine was obtained after 15 minutes.

Example 49

Operating proceeded as in Example 48, but by adding 1 g of p-nitrotoluene ($7.3\times10^{-3}$ moles).

After 20 minutes p-toluidine with a yield of >99% was obtained.

Ratios: S/C=730; KOH/C=1.

Example 50

Operating proceeded as described in Example 48, but by adding 2.78 g of p-nitrotoluene ($2\times10^{-2}$ moles).

After 120 minutes p-toluidine with a yield of >99% was obtained.

Ratios: S/C=2,000; KOH/C=1.

Example 51

Operating proceeded as described in Example 50, using 89.6 g of KOH. After 30 minutes 75% of p.p'-hydrazotoluene was obtained.

Ratios: S/C=2,000; KOH/cat.=80.

**Claims**

1. Process for the catalytic reduction of nitro-aromatic compounds through the transfer of hydrogen from alcohols to nitro-aromatic compounds, said reduction being catalyzed by complexes of iridium, characterized in that a primary or secondary alcohol having the formula (I) or a glycol:

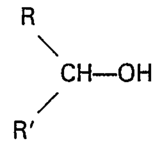

(I)

is made to react with a nitro-aromatic compound having the formula (II):

$$Ar—(NO_y)_x \qquad (II)$$

wherein:

R and R′, possibly linked to each other according to homo- or hetero-cycles, represent indifferently either a hydrogen atom or a hydrocarbylic group having up to 30 carbon atoms, possibly substituted;

Ar represents either an aryl or hetero-aryl group, possibly substituted with inert groups;

x and y, either equal or different from each other, represent an integer chosen from 1 to 2, in the presence of a complexed iridium catalyst chosen from among the catalysts having formulae (III) and (IV):

$$\{Ir\ Chel\ (L—L)\}X \qquad \text{and} \qquad Ir\ Chel\ (L)_2Y$$

(III) (IV)

Chel represents a kelating bi-toothed nitrogeneous compound;

L—L represents a diolefine molecule, preferably non conjugated;

L represents a monoolefine molecule;

$X^-$ represents an anion chosen from among $Cl^-$, $Br^-$, $I^-$, $PF_6^-$, $BF_4^-$, $ClO_4^-$, $B(C_6H_5)_4^-$, $OH^-$,

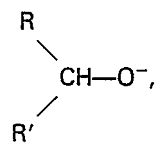

wherein

R and R′ have the significance already given to them;

Y is a halogen, preferably Cl, Br or I; and, optionally, in the presence of a compound chosen from mineral alkaline compounds at a temperature comprised between 20°C and the boiling temperature of the reaction mass.

2. Process according to claim 1, characterized in that the mineral alkaline compound is chosen from among NaOH, KOH, LiOH, $Ca(OH)_2$, $NaHCO_3$, according to a molar ratio with respect to the catalyst comprised between 0.1 and 200 moles per 1 mole of catalyst.

3. Process according to claim 1, characterized in that the primary or secondary alcohol of formula (I) defined in said claim, or the glycol, is substituted with groups inert at reaction conditions, preferably chosen from among the esteric, amidic, alcoxylic, aminic and cyano-groups.

4. Process according to claim 1, characterized in that the nitro-aromatic compound having the formula (II) therein defined consists of a nitro-compound derived from nitrous or nitric acid respectively for y=1 or 2, in formula (II), chosen from among the benzenic, naphtalenic, anthracenic, thienylic, furanic, pyridinic, quinolinic compounds, possibly substituted with groups inert under reaction conditions, preferably chosen from among the esteric, amidic, alcoxylic, aminic and cyano-groups and among alkylic, alkenylic and alkinic groups having up to 4 carbon atoms.

5. Process according to claim 1, characterized in that said process is conducted in a medium chosen between the alcohol or glycol donor in excess and a compound chosen from among toluene, benzene, methanol, water and terbutanol, also in admixture with each other, preferably in the alcohol or glycol donor.

6. Process according to claim 1, characterized in that the catalysts of formulae (III) and (IV) defined in said claim, are activated by means of molecular hydrogen and by the successive reflux heating in the reaction medium.

7. Process according to claim 1, characterized in that the kelating nitrogenous compound is chosen from among 2,2′-dipyridyl, 3,3′-dimethyl-2,2′-dipyridyl, 4,4′-dimethyl-2,2′-dipyridyl, 1,10-phenanthroline, 5,6-dimethyl-1,10-phenanthroline, 4,7-dimethyl-1,10-phenanthroline, 3,4,7,8-tetramethyl-1,10-phenanthroline, 4,7-diphenyl-1,10-phenanthroline, the sulphonated phenanthrolines.

8. Process according to claim 1, characterized in that the non-conjugated diolefine is preferably chosen from among 1,5-hexadiene, norbornadiene, 1,5-cyclooctadiene, and the mono-olefine is preferably chosen between cyclooctene and ethylene.

9. Process according to claim 1, characterized in that for 1 mole of nitro-aromatic (II) compound there are used from $1\times10^{-2}$ to $1\times10^{-4}$ moles of catalyst.

10. Process according to claim 1, characterized in that the complexed iridium catalyst is prepared 'in situ' in the reaction medium by the addition of the kelating compound to the halogenated olefinic iridium complex.

11. Process according to claim 1, characterized in that the molar ratio of the reactants alcohol or glycol donor/nitro-aromatic (II) compound is comprised between 1:1 and 200:1.

12. Process according to claim 1, characterized in that said process is conducted at a temperature comprised between 20° and 200°C, up to the boiling point of the reaction mass.

13. Process according to claim 1, characterized in that the said process is conducted at atmospheric pressure in an inert atmosphere, preferably chosen between nitrogen or argon.

14. Process according to claim 1, characterized in that the nitro-aromatic (II) compound therein defined is chosen from among: nitro-benzene, nitro-toluene, nitro-anisol, nitro-aniline, nitro-benzamide, nitrobenzonitrile, nitro-naphtalene, nitro-styrene, dinitro-benzene, dinitro-toluene, dinitro-aniline, dinitrobenzamide, dinitro-benzonitrile, nitro-pyridines, nitro-quinolines, nitroso-benzene.

15. Process according to claim 1, characterized in that the alcohol donor is chosen from among: isopropylic, ethylic, 2-butylic, benzylic, cyclohexylic and cyclopentylic alcohol.

16. Process according to claim 1, characterized in that the catalyst of either formula (III) or (IV) therein defined, is chosen from among:

. Ir 3,4,7,8$(CH_3)_4$phen$(CH_2{=}CH_2)_2$Cl;
. Ir 4,7$(CH_3)_2$phen$(CH_2{=}CH_2)_2$Cl;
. Ir phen$(CH_2{=}CH_2)_2$Cl;
. Ir 4,7$(C_6H_5)_2$phen$(CH_2{=}CH_2)_2$Cl;
. Ir 4,4'$(CH_3)_2$dipy$(CH_2{=}CH_2)_2$Cl;
. Ir 4,4'$(C_6H_5)_2$dipy$(CH_2{=}CH_2)_2$Cl;
. Ir$(C_6H_5)_2$dipy$(CH_2{=}CH_2)_2$Cl;
. Ir dipy$(CH_2{=}CH_2)_2$Cl;
. Ir 3,4,7,8$(CH_3)_4$phen COD Cl;
. {Ir 3,4,7,8$(CH_3)_4$phen(1,5-hexadiene)}$ClO_4$;
. {Ir 4,7$(CH_3)_2$phen(1,5-hexadiene)}$ClO_4$;
. {Ir phen(1,5-hexadiene)}$ClO_4$;
. {Ir 4,7$(C_6H_5)_2$phen(1,5-hexadiene)}$ClO_4$;
. {Ir 4,4'$(CH_3)_2$dipy(1,5-hexadiene)}$ClO_4$;
. {Ir 4,4'$(C_6H_5)_2$dipy(1,5-hexadiene)}$ClO_4$;
. {Ir dipy(1,5-hexadiene)}$ClO_4$;

wherein:

"phen" stands for phenanthroline;
"dipy" for 2,2'-dipyridyl;
"COD" for 1,5-cyclooctadiene.

17. Process according to claim 1, characterized in that as starting aromatic compound is used a derivative from the partial hydrogenation of the nitro-aromatic compounds of formula (II) therein defined, chosen from among the azoxy- and the azo-derivatives of the same.

18. Process according to claim 1, characterized in that the nitro-aromatic compound of formula (II) therein defined is selectively reduced to the corresponding amine using a molar ratio of alkaline compound/catalyst comprised between 0.5:1 and 1.5:1, and a molar ratio nitro-aromatic compound (II)/catalyst comprised between 5:1 and 250:1.

19. Process according to claim 1, characterized in that the nitro-aromatic compound of formula (II) therein defined is selectively reduced to the corresponding hydrazo-derivative, using a molar ratio alkaline compound/catalyst comprised between 10:1 and 100:1, and a molar ratio nitro-aromatic (II) compound/catalyst comprised between 500:1 and 2000:1.

20. Process according to claim 1, characterized in that the nitro-aromatic compound of formula (II) therein defined is selectively reduced to the corresponding hydroxylamine derivative, using a nitro-aromatic (II) compound/catalyst molar ratio comprised between 50:1 and 250:1, and an alkaline compound/catalyst molar ratio comprised between 1:1 and 10:1, and by blocking the reaction at the desired stage.

21. Process according to claims 18 and 19, characterized in that the reduction of the nitro-aromatic (II) compounds to the corresponding amino- and hydrazo-derivatives, is preferably carried out in the presence of a catalyst of either formula (IV) or (III), as defined in claim 1, and chosen from between: Ir 3,4,7,8$(CH_3)_4$phen$(CH_2{=}CH_2)_2$Cl and {Ir 3,4,7,8$(CH_3)_4$phen (1,5-hexadiene)}$ClO_4$ wherein "phen" stands for phenanthroline.

22. Process according to claims 18 and 20, characterized in that the reduction of the nitro-aromatic (II)

compounds to the corresponding amino- and hydroxylamino-derivatives is preferably carried out in the presence of a catalyst of either formula (III) or (IV), defined in claim 1, chosen from among:

. Ir $4,4'(C_6H_5)_2dipy(CH_2{=}CH_2)Cl$;
. Ir $4,7(C_6H_5)_2phen(CH_2{=}CH_2)Cl$;
. Ir $4,4'(C_6H_5)_2dipy(1,5\text{-hexadiene})ClO_4$;
. Ir $4,7(C_6H_5)_2phen(1,5\text{-hexadiene})ClO_4$;

wherein:
"dipy" stands for 2,2'-dipyridyl; and,
"phen" stands for phenanthroline.

23. Process according to claim 1, characterized in that for x=2 in formula (II) of the therein defined nitro-aromatic compound, the reduction reaction is conducted using an alkaline compound/catalyst molar ratio equal to 1:1 and a substrate/catalyst molar ratio below 10.

**Patentansprüche**

1. Verfahren zum katalytischen Reduzieren von nitroaromatischen Verbindungen durch Anlagern von von Alkohol herrührendem Wasserstoff an nitroaromatische Verbindungen, bei welchem die Reduktion durch Iridium-komplexe katalysiert wird, dadurch gekennzeichnet, dass man einen primären oder sekundären Alkohol gemäss der Formel (I) oder ein Glykol:

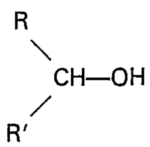

(I)

mit einer nitroaromatischen Verbindung gemäss der Formel (II) reagieren lässt:

$$AR—(NO_y)_x \qquad (II)$$

worin:

R und R', gegebenenfalls homozyklisch oder heterozyklisch miteinander verbunden, je entweder ein Wasserstoffatom oder eine ggf. substituierte, bis zu 30 Kohlenstoffatome enthaltende Kohlenwasserstoffgruppe darstellen;

Ar entweder eine Arylgruppe oder eine Heteroarylgruppe darstellt, die ggf. durch inerte Gruppen substituiert ist;

x und y, die gleiche oder verschiedene Werte besitzen können, eine ganze Zahl darstellen, die entweder gleich 1 oder gleich 2 ist, wobei man diese Reaktion in Gegenwart eines komplexen Iridiumkatalysators durchführt, der zu der Gruppe der Katalysatoren gemäss der Formeln (III) und (IV) gehört:

$$\{Ir\ Chel\ (L—L)\}X^- \qquad (III)$$

und

$$Ir\ Chel\ (L)_2Y \qquad (IV)$$

worin:

Chel eine doppelzweigige chelatbildende Stickstoffverbindung darstellt;
L—L ein vorzugsweise nicht konjugiertes Diolefin-Molekül darstellt;
L ein Monoolefin-Molekül darstellt;
$X^-$ ein Anion der Gruppe:
$Cl^-$, $Br^-$, $I^-$, $PF_6^-$, $BF_4^-$, $ClO_4^-$, $B(C_6H_5)_4^-$, $OH^-$,

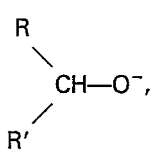

worin:

R und R' die obige Bedeutung haben;
Y ein Halogen ist, und zwar vorzugsweise Cl, Br oder I;

während die Reaktion gegebenenfalls auch in Gegenwart einer anorganischen Alkaliverbindung bei einer Temperatur durchgeführt wird, die zwischen 20°C und dem Siedepunkt der Reaktionsmasse liegt.

2. Verfahren nach Anspruch 1, dadurch gekennzeichnet, dass die anorganische Alkaliverbindung nach Massgabe eines Molverhältnisses in bezug auf den Katalysator derart aus der Gruppe NaOH, KOH, LiOH, $Ca(OH)_2$, $NaHCO_3$ gewählt wird, dass die 0,1 Mol bis 200 Mol pro Mol Katalysator ausmacht.

3. Verfahren nach Anspruch 1, dadurch gekennzeichnet, dass der primäre oder sekundäre Alkohol der Formel (I) gemäss des genannten Anspruchs bzw. das Glykol durch unter Reaktionsbedingungen inerte Gruppen, und zwar vorzugsweise durch Ester=, Amid=, Alkoxyl=, Amin= und Zyangruppen substituiert ist.

4. Verfahren nach Anspruch 1, dadurch gekennzeichnet, dass die der im genannten Anspruch definierten Formel (II) entsprechende nitroaromatische Verbindung eine für y=1 oder 2 in Formel (II) von Salpetrigsäure bzw. Salpetersäure hergeleitete stickstoffhaltige Verbindung ist, die zur folgenden Gruppe gehört: Benzol=, Naphthol=, Anthracen=, Thienyl=, Furan=, Pyridin= und Chinolinverbindungen, ggf. durch unter Reaktionsbedingungen inerte Gruppen substituiert, die vorzugsweise Ester=, Amid=, Alkoxyl=, Amin bzw. Zyangruppen oder Alkyl=, Alkenyl= und Alkingruppen mit höchstens 4 Kohlenstoffatomen sind.

5. Verfahren nach Anspruch 1, dadurch gekennzeichnet, dass es in einem Medium durchgeführt wird, das besteht aus dem im Überschuss vorliegenden Alkohol oder dem Glykol und Toluol, Benzol, Methanol, Wasser oder Terbutanol, ggf. miteinander und vorzugsweise im Alkohol oder Glykol gemischt.

6. Verfahren nach Anspruch 1, dadurch gekennzeichnet, dass die Katalysatoren gemäss der im genannten Anspruch definierten Formeln (III) und (IV) durch molekularen Wasserstoff und durch wiederholtes Erwärmen im Rückfluss innerhalb des Reaktionsmediums aktiviert werden.

7. Verfahren nach Anspruch 1, dadurch gekennzeichnet, dass die chelatbildende stickstoffhaltige Verbindung aus folgender Gruppe gewählt wird: 2,2'-Dipyridyl (dipy), 3,3'-Dimethyl-2,2'-dipyridyl, 4,4'-Dimethyl-2,2'-dipyridyl, 1,10-Phenanthrolin, 5,6-Dimethyl-1,10-phenanthrolin, 4,7-Dimethyl-1,10-phenanthrolin, 3,4,7,8-Tetramethyl-1,10-phenanthrolin, 4,7-Diphenyl-1,10-phenanthrolin und sulfoniertes Phenanthrolin.

8. Verfahren nach Anspruch 1, dadurch gekennzeichnet, dass das nicht konjugierte Diolefin vorzugsweise, 1,5-Hexadien, Norbornadien oder, 1,5-Zyclooctadien ist, während das Monoolefin vorzugsweise Zyclookten oder Athylen ist.

9. Verfahren nach Anspruch 1, dadurch gekennzeichnet, dass pro Mol nitroaromatischer Verbindung (II) $1\times10^{-2}$ bis $1\times10^{-4}$ Mol Katalysator eingesetzt wird.

10. Verfahren nach Anspruch 1, dadurch gekennzeichnet, dass der komplexe Iridiumkatalysator extemporan im Reaktionsmedium durch Beigabe der chelatbildenden Verbindung zum halogenolefinischen Iridiumkomplex erzeugt wird.

11. Verfahren nach Anspruch 1, dadurch gekennzeichnet, dass das Mol-Verhältnis "Alkohol bzw. Glykol/nitroaromatische Verbindung (II)" zwischen 1:1 und 200:1 liegt.

12. Verfahren nach Anspruch 1, dadurch gekennzeichnet, dass es bei einer Temperatur durchgeführt wird, die zwischen 20° und 200°C, bis zum Siedepunkt der Reaktionsmasse liegt.

13. Verfahren nach Anspruch 1, dadurch gekennzeichnet, dass es bei atmosphärischem Druck in einer inerte Atmosphäre, wie vorzugsweise Stickstoff= oder Argonatmosphäre durchgeführt wird.

14. Verfahren nach Anspruch 1, dadurch gekennzeichnet, dass die im genannten Anspruch definierte nitroaromatische Verbindung (II) eine der folgenden Verbindungen ist: Nitrobenzol, Nitrotoluol, Nitroanisol, Nitroanilin, Nitrobenzamid, Nitrobenzonitril, Nitronaphtol, Nitrostyren, Dinitrobenzol, Dinitrotoluol, Dinitroanilin, Dinitrobenzamid, Dinitrobenzonitril, Nitropyridin, Nitrochinolin, Nitrobenzol.

15. Verfahren nach Anspruch 1, dadurch gekennzeichnet, dass der Alkohol Isopropyl=, Äthyl=, 2-Butyl=, Benzyl=, Zyklohexyl= oder Zyklopentylalkohol ist.

16. Verfahren nach Anspruch 1, dadurch gekennzeichnet, dass der in Formel (III) oder (IV) des genannten Anspruchs definierte Katalysator aus folgender Gruppe gewählt wird:

. Ir 3,4,7,8$(CH_3)_4$phen$(CH_2{=}CH_2)_2$Cl;
. Ir 4,7$(CH_3)_2$phen$(CH_2{=}CH_2)_2$Cl;
. Ir phen$(CH_2{=}CH_2)_2$Cl;
. Ir 4,7$(C_6H_5)_2$phen$(CH_2{=}CH_2)_2$Cl;
. Ir 4,4'$(CH_3)_2$dipy$(CH_2{=}CH_2)_2$Cl;
. Ir 4,4'$(C_6H_5)_2$dipy$(CH_2{=}CH_2)_2$Cl;
. Ir $(C_6H_5)_2$dipy$(CH_2{=}CH_2)_2$Cl;
. Ir dipy$(CH_2{=}CH_2)_2$Cl;
. Ir 3,4,7,8$(CH_3)_4$phen COD Cl;
. {Ir 3,4,7,8$(CH_3)_4$)phen(1,5-Hexadien)}$ClO_4$;
. {Ir 4,7$(CH_3)_2$phen(1,5-Hexadien)}$ClO_4$;
. {Ir phen(1,5-Hexadien)}$ClO_4$;
. {Ir 4,7$(C_6H_5)_2$phen(1,5-Hexadien)}$ClO_4$;
. {Ir 4,4'$(CH_3)_2$dipy(1,5-Hexadien)}$ClO_4$;
. {Ir 4,4'$(C_6H_5)_2$dipy(1,5-Hexadien)}$ClO_4$;
. {Ir dipy(1,5-Hexadien)}$ClO_4$;

worin:
"phen" Phenanthrolin darstellt;
"dipy" 2,2'-Dipyridyl darstellt; und
"COD" 1,5-Zyklooctadien darstellt.

17. Verfahren nach Anspruch 1, dadurch gekennzeichnet, dass als aromatische Ausgangsverbindung

ein durch teilweise Hydrierung erzeugtes Derivat der nitroaromatischen Verbindungen gemäss der im genannten Anspruch definierten Formel (II) verwendet, das zu den Azoxy= oder Azoderivaten derselben gehört.

18. Verfahren nach Anspruch 1, dadurch gekennzeichnet, dass die nitroaromatische Verbindung gemäss der im genannten Anspruch definierten Formel (II) wahlweise zu dem entsprechenden Amin reduziert wird, und zwar nach Massgabe eines Mol-Verhältnisses "Alkaliverbindung/Katalysator" von 0,5:1 bis 1,5:1 und eines Mol-Verhältnisses "nitroaromatische Verbindung (II)/Katalysator" von 5:1 bis 250:1.

19. Verfahren nach Anspruch 1, dadurch gekennzeichnet, dass die nitroaromatische Verbindung gemäss der im genannten Anspruch definierten Formel (II) wahlweise zu dem entsprechenden Hydrazo-Derivat reduziert wird, nach Massgabe eines Mol-Verhältnisses "Alkali= Verbindung (II)/Katalysator" von 10:1 bis 100:1, und eines Mol-Verhältnisses "nitroaromatische Verbindung (II)/Katalysator" von 500:1 bis 2000:1.

20. Verfahren nach Anspruch 1, dadurch gekennzeichnet, dass die nitroaromatische Verbindung gemäss der im genannten Anspruch definierten Formel (II) wahlweise zum entsprechenden Hydroxylamin-Derivat reduziert wird, nach Massgabe eines Mol-Verhältnisses "nitroaromatische Verbindung (II)/Katalysator" von 50:1 bis 250:1, und eines Mol-Verhältnisses "Alkaliverbindung/Katalysator" von 1:1 bis 10:, wobei die Reaktion im geeigneten Stadium unterbrochen wird.

21. Verfahren nach den Ansprüchen 18 und 19, dadurch gekennzeichnet, dass die Reduktion der nitroaromatischen Verbindungen (II) zu den entsprechenden Amino= und Hydrazoderivaten vorzugsweise in Gegenwart eines Katalysators gemäss der in Anspruch 1 definierten Formel (IV) oder (III) erfolgt, welcher aus Ir $3,4,7,8(CH_3)_4phen(CH_2{=}CH_2)_2Cl$ oder aus {Ir $3,4,7,8(CH_3)_4$phen(1,5-Hexadien)}$ClO_4$ besteht, wobei "phen" Phenanthrolin bedeutet.

22. Verfahren nach den Ansprüchen 18 und 20, dadurch gekennzeichnet, dass die Reduktion der nitroaromatischen Verbindungen (II) zu den entsprechenden Amino= und Hydroxylaminoderivaten vorzugsweise in Gegenwart eines Katalysators gemäss der in Anspruch 1 definierten Formel (III) oder (IV) erfolgt, der aus folgender Gruppe gewählt wird:

. Ir $4,4'(C_6H_5)_2dipy(CH_2{=}CH_2)_2Cl$;
. Ir $4,7(C_6H_5)_2phen(CH_2{=}CH_2)_2Cl$;
. {Ir $4,7(C_6H_5)_2$phen(1,5-Hexadien)}$ClO_4$;
. {Ir $4,4'(C_6H_5)_2$dipy(1,5-Hexadien)}$ClO_4$;

worin:

"dipy" 2,2-Dipyridyl und
"phen" Phenanthrolin darstellt.

23. Verfahren nach Anspruch 1, dadurch gekennzeichnet, dass bei x=2 in der im genannten Anspruch definierten Formel (II) der nitroaromatischen Verbindung die Reduktionsreaktion mit einem Mol-Verhältnis "Alkaliverbindung/Katalysator" von 1:1 und einem Mol-Verhältnis "Substrat/Katalysator" von weniger als 10 durchgeführt wird.

**Revendications**

1. Procédé de réduction catalytique de composés nitro-aromatiques par transfert d'hydrogène à partir d'alcools vers des composés nitro-aromatiques, ladite réduction étant catalysée par des complexes d'iridium, caractérisé en ce qu'il consiste à faire réagir un alcool primaire ou secondaire de formule (I) ou un glycol:

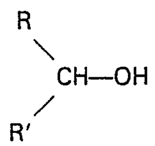

(I)

avec un composé nitro-aromatique de formule (II):

$$AR{-}(NO_y)_x \qquad (II)$$

où:

R et R', éventuellement liés l'un à l'autre, homocycliquement ou hétérocycliquement, représentent indifféremment soit un atome d'hydrogène, soit un groupe hydrocarbyle renfermant jusqu'à 30 atomes de carbone et éventuellement substitué;

Ar représente soit un groupe aryle, soit un groupe hétéroaryle éventuellement substitué par des groupes inertes;

x et y, égaux ou différents, représentent chacun un nombre entier égal à 1 ou à 2; en présence d'un catalyseur à base de complexe d'iridium qui correspond, soit à la formule (III), soit la formule (IV):

$$\{Ir\ Chel\ (L{-}L)\}X^- \qquad (III)$$

$$Ir\ Chel\ (L)_2Y \qquad (IV)$$

où:

Chel représente un composé nitro-aromatique de chélation à deux branches;

L—L représente une molécule de dioléfine, de préférence non conjuguée;

L représente une molécule de mono-oléfine;

X représente un anion choisi parmi les suivants:

$Cl^-$, $Br^-$, $I^-$, $PF_6^-$, $BF_4^-$, $ClO_4^-$, $B(C_6H_5)_4^-$, $OH^-$,

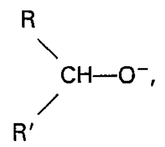

où:

R et R′ ont la signification ci-dessus;

Y est un halogène, de préférence Cl, Br ou I; et à conduire cette réaction éventuellement en présence d'un composé choisi parmi les composés minéraux alcalins à une température comprise entre 20°C et le point d'ébullition de la masse réactive.

2. Procédé selon la revendication 1, caractérisé en ce que la composé minéral alcalin est choisi parmi NaOH, KOH, LiOH, $Ca(OH)_2$, $NaHCO_3$ selon un rapport molaire par référence au catalyseur, qui est compris entre 0,1 et 2000 moles par mole de catalyseur.

3. Procédé selon la revendication 1, caractérisé en ce que l'alcool primaire ou secondaire de la formule (I) définie dans ladite revendication, ou le glycol, sont substitués par des groupes qui sont inertes dans les conditions de réaction et qui sont choisis, de préférence, parmi les groupes esters, amides, alcoxyles, amines et cyano.

4. Procédé selon la revendication 1, caractérisé en ce que le composé nitro-aromatique selon la formule (II) définie dans ladite revendication est constitué par un composant azoté dérivé de l'acide nitreux ou de l'acide nitrique pour y=1 ou y=2, respectivement, dans la formule (II), ce composé étant choisi parmi ceux qui forment le groupe comprenant les composés à base de benzène, naphtalène, anthracène, thiényle, furanne, pyridine, quinoline, éventuellement substitués par des groupes inertes dans les conditions de réaction et choisis de préférence parmi les groupes esters, amides, alcoxydes, amines et cyanos et parmi les groupes alkyles, alkényles et alkynes renfermant jusqu'à 4 atomes de carbone.

5. Procédé selon la revendication 1, caractérisé en ce qu'il est mis en oeuvre dans un milieu constitué par un excès d'alcool ou de glycol et un composé du groupe toluène, benzène, méthanol, eau et tertiobutanol, éventuellement mélangés, de préférence dans l'alcool ou le glycol.

6. Procédé selon la revendication 1, caractérisé en ce que les catalyseurs de formule (III) et de formule (IV) définies dans ladite revendication sont activés par de l'hydrogène moléculaire et par le réchauffage ultérieur à reflux dans le milieu réactif.

7. Procédé selon la revendication 1, caractérisé en ce que le composé azoté de chélation est un composé du groupe comprenant: 2,2′-diméthyl-2,2′-dipyridyle, 4,4′-diméthyl-2,2′-dipyridyle, 1,10-phénanthroline, 4,6-diméthyl-1,10-phénanthroline, 4,7-diméthyl-1,10-phénanthroline, 3,4,7,8-tétraméthyl-1,10-phénanthroline, 4,7-diphényl-1,10-phénanthroline et les phénanthrolines sulfonatées.

8. Procédé selon la revendication 1, caractérisé en ce que la dioléfine non conjuguée est choisie, de préférence, parmi les composés du groupe 1,5-hexadiène, norbornadiène, 1,5-cyclooctadiène, tandis que la mono-oléfine est, de préférence, du cyclooctène ou de l'éthylène.

9. Procédé selon la revendication 1, caractérisé en ce qu'on utilise pour une mole de composé nitro-aromatique (II) $1\times10^{-2}$ à $1\times10^{-4}$ de catalyseur.

10. Procédé selon la revendication 1, caractérisé en ce qu'on prépare le catalyseur complexe à base d'iridium in situ dans le milieu réactif en ajoutant le composé de chélation au composé complexe à base d'oléfine et d'iridium.

11. Procédé selon la revendication 1, caractérisé en ce que le rapport molaire alcool/composé nitro-aromatique (II) ou glycol/composé nitro-aromatique (II) est compris entre 1/1 et 200/1.

12. Procédé selon la revendication 1, caractérisé en ce qu'il est mis en oeuvre à une température comprise entre 20°C et 200°C jusqu'à concurrence du point d'ébullition de la masse réactive.

13. Procédé selon la revendication 1, caractérisé en ce qu'il est mis en oeuvre sous la pression atmosphérique dans une atmosphère inerte formée, de préférence, d'azote ou d'argon.

14. Procédé selon la revendication 1, caractérisé en ce que le composé nitro-aromatique (II) défini dans ladite revendication est choisi du groupe comprenant le: nitrobenzène, nitrotoluène, nitroanisol, nitroaniline, nitrobenzamide, nitrobenzonitrile, nitronaphtalène, nitrostyrène, dinitrobenzène, dinitrotoluène, dinitroaniline, dinitrobenzamide, dinitrobenzonitrile, les nitropyridines, les nitroquinolines et le nitrosobenzène.

15. Procédé selon la revendication 1, caractérisé en ce que ledit alcool est chois dans le groupe comprenant les alcools isopropylique, éthylique, 2-butylique, benzylique, cyclohexylique et cyclopentylique.

16. Procédé selon la revendication 1, caractérisé en ce que le catalyseur selon la formule (III) ou (IV) définies dans ladite revendication est choisi dans le groupe comprenant:

. Ir 3,4,7,8$(CH_3)_4$phèn$(CH_2{=}CH_2)_2$Cl;
. Ir 4,7$(CH_3)_2$phèn$(CH_2{=}CH_2)_2$Cl;
. Ir phèn$(CH_2{=}CH_2)_2$Cl;
. Ir 4,7$(C_6H_5)_2$phèn$(CH_2{=}CH_2)_2$Cl;
. Ir 4,4'$(CH_3)_2$dipy$(CH_2{=}CH_2)_2$Cl;
. Ir 4,4'$(C_6H_5)_2$dipy$(CH_2{=}CH_2)_2$Cl;
. Ir$(C_6H_5)_2$dipy$(CH_2{=}CH_2)_2$Cl;
. Ir dipy$(CH_2{=}CH_2)_2$Cl;
. Ir 3,4,7,8$(CH_3)_4$phèn COD Cl;
. {Ir 3,4,7,8$(CH_3)_4$phèn(1,5-hexadiène)}$ClO_4$;
. {Ir 4,7$(CH_3)_2$phèn(1,5-hexadiène)}$ClO_4$;
. {Ir phèn(1,5-hexadiène)}$ClO_4$;
. {Ir 4,7$(C_6H_5)_2$phèn(1,5-hexadiène)}$ClO_4$;
. {Ir 4,4'$(CH_3)_2$dipy(1,5-hexadiène)}$ClO_4$;
. {Ir 4,4'$(C_6H_5)_2$dipy(1,5-hexadiène)}$ClO_4$;
. {Ir dipy(1,5-hexadiène)}$ClO_4$;

où:

phèn représente la phénanthroline;
dipy représente le 2,2'-dipyridyle; et
COD représente le 1,5-cyclooctadiène.

17. Procédé selon la revendication 1, caractérisé en ce qu'on utilise comme composé aromatique de départ un dérivé obtenu par hydrogénation partielle des composés nitro-aromatiques selon la formule (II) définie dans ladite revendication, ce dérivé faisant partie du groupe des dérivés azoxy et azo des composés précités.

18. Procédé selon la revendication 1, caractérisé en ce que le composé nitro-aromatique selon la formule (II) définie dans ladite revendication est transformé par réduction sélective en l'amine correspondante, cependant qu'on prévoit un rapport molaire de composé alcalin/catalyseur compris entre 0,5/1 et 1,1/1 et un rapport molaire composé nitro-aromatique (II)/catalyseur compris entre 5/1 et 250/1.

19. Procédé selon la revendication 1, caractérisé en ce que le composé nitro-aromatique selon la formule (II) définie dans ladite revendication est transformé par réduction sélective en son dérivé hydrazo correspondant, cependant qu'on prévoit un rapport molaire composé alcalin/catalyseur compris entre 10/1 et 100/1 et un rapport molaire composé nitro-aromatique (II)/catalyseur compris entre 500/1 et 2000/1.

20. Procédé selon la revendication 1, caractérisé en ce que le composé nitro-aromatique selon la formule (II) définie dans ladite revendication est transformé par réduction sélective en un dérivé hydroxylamine correspondant, cependant qu'on prévoit un rapport molaire composé nitro-aromatique (II)/catalyseur compris entre 50/1 et 250/1 et un rapport molaire composé alcalin/catalyseur compris entre 1/1 et 10/1, et que l'on bloque la réaction au stade voulu.

21. Procédé selon les revendications 18 et 19, caractérisé en ce que la transformation par réduction des composés nitro-aromatiques (II) en dérivés amino et hydrazo correspondants est effectuée de préférence en présence d'un catalyseur de formule (IV) ou de formule (III) telles que définies dans la revendication 1, ce catalyseur étant choisi dans le groupe comprenant Ir 3,4,7,8$(CH_3)_4$phèn$(CH_2{=}CH_2)_2$Cl et {Ir 3,4,7,8$(CH_3)_4$phèn(1,5-hexadiène)}$ClO_4$; où phèn représente la phénanthroline.

22. Procédé selon les revendications 18 et 20, caractérisé en ce que la transformation par réduction des composés nitro-aromatiques (II) en dérivés amino et hydroxylamine correspondants est effectuée, de préférence, en présence d'un catalyseur de formule (III) ou de formule (IV) telles que définies dans la revendication 1, ce catalyseur étant choisi dans le groupe comprenant:

. Ir 4,4'$(C_6H_5)_2$dipy$(CH_2{=}CH_2)_2$Cl;
. Ir 4,7$(C_6H_5)_2$phèn$(CH_2{=}CH_2)_2$Cl;
. {Ir 4,7$(C_6H_5)_2$phèn(1,5-hexadiène)}$ClO_4$;
. {Ir 4,4'$(C_6H_5)_2$dipy(1,5-hexadiène)}$ClO_4$;

où:

dipy représente le 2,2'-dipyridyle;
phèn représente la phénanthroline.

23. Procédé selon la revendication 1, caractérisé en ce que, pour x=2 dans la formule (II) du composé nitro-aromatique qui y est défini, la réaction de réduction est effectuée avec un rapport molaire composé alcalin/catalyseur égal à 1/1 et un rapport moliare substrat/catalyseur inférieur à 10.